# DEMANDE DE BREVET EUROPEEN

(11) **EP 4 442 373 A1**
(43) Date de publication de la demande: **09.10.2024**
(21) Numéro de dépôt: 24166112.3
(22) Date de dépôt: 26.03.2024
(51) Int. Cl.: B06B 1/02, A61B 8/00, B06B 1/06, H05K 1/18

(54) **PROCEDE DE FABRICATION D'UNE SONDE ULTRASONORE**

(30) Priorité: 03.04.2023 FR 2303295
(71) Demandeur: Vermon, 37000 Tours (FR)
(72) Inventeur: MEYNIER, Cyril, 37000 Tours (FR)
(74) Mandataire: Cabinet Beaumont

(57) **Abrégé**

La présente description concerne un procédé de fabrication d'une sonde ultrasonore, comprenant les étapes suivantes :
a) rapporter, sur un substrat d'interconnexion flexible (101), une puce (201) comportant une pluralité de transducteurs ultrasonores, la puce (201) comportant une face avant comportant au moins un plot de connexion électrique (203) et une face arrière ;
b) replier une partie du substrat d'interconnexion flexible (101), de façon que le substrat d'interconnexion flexible (101) s'étende sur au moins une partie de la face arrière de la puce et sur au moins une partie de la face avant de la puce, ledit au moins un plot de connexion électrique (203) de la face avant de la puce étant connecté à au moins un plot de connexion électrique (205) correspondant du substrat d'interconnexion ; et
c) découper la puce (201) en une pluralité de puces élémentaires (103) comportant chacune un ou plusieurs transducteurs ultrasonores.

## Description

### Domaine technique

La présente description concerne de façon générale la fabrication de sondes ultrasonores, et vise en particulier la fabrication de sondes ultrasonores de petites dimensions destinées à être montées sur un cathéter, par exemple pour des applications de diagnostic ou de traitement ultrasonore intravasculaire.

### Technique antérieure

On a déjà proposé d'intégrer des sondes ultrasonores de petites dimensions à un cathéter destiné à être introduit dans le corps d'un patient humain ou animal, par exemple pour des applications de diagnostic ou de traitement ultrasonore intravasculaire.

Il serait souhaitable de pouvoir disposer d'un procédé de fabrication d'une telle sonde, ce procédé palliant au moins en partie certains des inconvénients des procédés connus.

### Résumé de l'invention

Un mode de réalisation prévoit un procédé de fabrication d'une sonde ultrasonore, comprenant les étapes suivantes :
a) rapporter, sur un substrat d'interconnexion flexible, une puce comportant une pluralité de transducteurs ultrasonores, la puce comportant une face avant comportant au moins un plot de connexion électrique et une face arrière ;
b) replier une partie du substrat d'interconnexion flexible, de façon que, à l'issue de l'étape b), le substrat d'interconnexion flexible s'étende sur au moins une partie de la face arrière de la puce et sur au moins une partie de la face avant de la puce, ledit au moins un plot de connexion électrique de la face avant de la puce étant connecté à au moins un plot de connexion électrique correspondant du substrat d'interconnexion ; et
c) découper la puce en une pluralité de puces élémentaires comportant chacune un ou plusieurs transducteurs ultrasonores.

Selon un mode de réalisation, lequel l'étape c) est mise en oeuvre après l'étape b).

Selon un mode de réalisation, l'étape c) est mise en oeuvre avant l'étape b).

Selon un mode de réalisation, à l'étape a), la puce a sa face arrière tournée vers la face du substrat d'interconnexion comportant ledit au moins un plot de connexion électrique du substrat d'interconnexion.

Selon un mode de réalisation, à l'étape a), la puce a sa face avant tournée vers la face du substrat d'interconnexion comportant ledit au moins un plot de connexion électrique du substrat d'interconnexion.

Selon un mode de réalisation, à l'issue de l'étape c), les puces élémentaires restent reliées mécaniquement les unes aux autres par une bande non découpée du substrat d'interconnexion flexible.

Selon un mode de réalisation, lors de l'étape b), une tige cylindrique est positionnée sur le substrat d'interconnexion pour contrôler la courbure du substrat d'interconnexion lors du pliage.

Selon un mode de réalisation, le procédé comprend, après les étapes a), b) et c), une étape de courbure du substrat d'interconnexion selon une forme souhaitée de la sonde, par exemple une forme cylindrique.

Selon un mode de réalisation, les transducteurs ultrasonores sont des transducteurs de type CMUT ou PMUT.

Selon un mode de réalisation, la puce comporte en outre au moins un plot de connexion électrique du côté de sa face arrière.

Un autre mode de réalisation prévoit une sonde ultrasonore comportant un substrat d'interconnexion flexible et une pluralité de puces élémentaires fixées au substrat d'interconnexion flexible, chaque puce élémentaire comportant un ou plusieurs transducteurs ultrasonores, chaque puce élémentaire comportant une face avant comportant au moins un plot de connexion électrique et une face arrière,
dans laquelle une partie du substrat d'interconnexion flexible est replié sur les puces élémentaires, de façon que le substrat d'interconnexion flexible s'étende sur au moins une partie de la face arrière de chaque puce élémentaire et sur au moins une partie de la face avant de chaque puce élémentaire, ledit au moins un plot de connexion électrique de la face avant de chaque puce élémentaire étant connecté à un plot de connexion électrique correspondant du substrat d'interconnexion.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 est une vue en perspective très schématique d'un exemple d'une sonde ultrasonore ;
la figure 2A, la figure 2B et la figure 2C illustrent des étapes d'un exemple d'un procédé de fabrication d'une sonde ultrasonore selon un mode de réalisation ; et
la figure 3A et la figure 3B illustrent des étapes d'une variante d'un procédé de fabrication d'une sonde ultrasonore selon un mode de réalisation.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, la réalisation des transducteurs ultrasonores des sondes décrites n'a pas été détaillée, les modes de réalisation décrits étant compatibles avec toutes ou la plupart des structures connues de transducteurs ultrasonores. En outre, la réalisation des circuits de commande des transducteurs ultrasonores n'a pas été détaillée, les modes de réalisation décrits étant compatibles avec les circuits de commande usuels de transducteurs ultrasonores ou la réalisation des circuits de commande étant à la portée de la personne du métier à partir des indications de la présente description.

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des figures.

Sauf précision contraire, les expressions "environ", "approximativement", "sensiblement", et "de l'ordre de" signifient à 10 % près, de préférence à 5 % près.

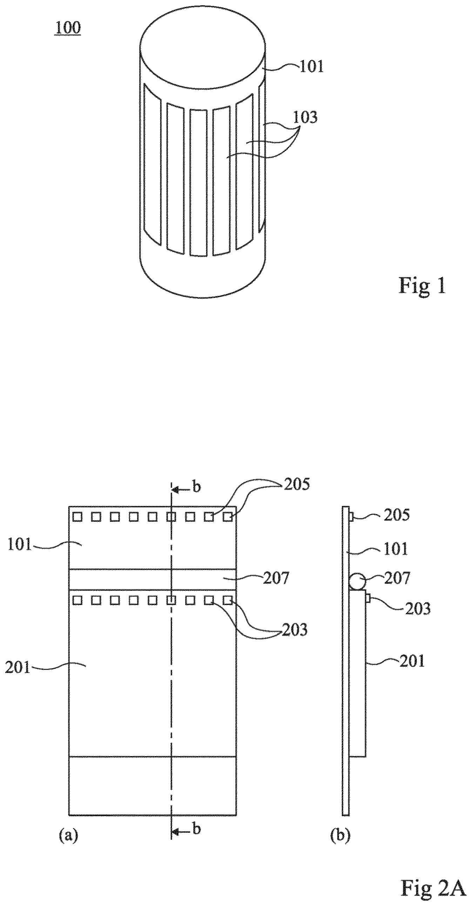

La figure 1 est une vue en perspective très schématique d'un exemple d'une sonde ultrasonore 100.

La sonde 100 comprend un substrat d'interconnexion flexible 101 sur lequel sont fixées une pluralité de puces élémentaires 103 comportant chacune un ou plusieurs transducteurs ultrasonores, non détaillés sur les figures. A titre d'exemple, chaque puce élémentaire 103 est une puce d'émission/réception d'ondes ultrasonores, aussi appelée "barrette ultrasonore", composée d'un ou plusieurs transducteurs ultrasonores élémentaires, par exemple alignés selon un axe parallèle à un axe longitudinal de la sonde ou disposés en matrice sur le plan de la barrette.

Le substrat d'interconnexion flexible 101 est enroulé autour d'un cylindre (non visible sur la figure), par exemple à section circulaire, de façon à obtenir une sonde de forme générale cylindrique, par exemple de petites dimensions, par exemple de diamètre compris entre 1 et 5 mm. Les puces élémentaires forment des facettes adaptées à émettre des ultrasons vers l'extérieur du cylindre, dans des directions radiales. Dans cet exemple, les puces élémentaires ont la forme de bandes rectangulaires parallèles, s'étendant parallèlement à l'axe central du cylindre. A titre d'exemple, la sonde comprend au moins deux, de préférence au moins quatre, de préférence au moins huit, puces élémentaires 103, par exemple régulièrement réparties autour du cylindre.

Chaque puce comprend des plots de connexion électrique (non visibles sur la figure 1) connectés à des éléments de connexion électrique correspondants (non visibles sur la figure 1) du substrat d'interconnexion 101.

Le substrat d'interconnexion 101 est par exemple un circuit imprimé flexible, comportant des éléments d'interconnexion électriquement conducteurs, par exemple des pistes et plots métalliques, par exemple en cuivre, formés sur un support diélectrique souple, par exemple en un matériau polymère, par exemple un polyimide. Un ou plusieurs fils électriquement conducteurs, non détaillés sur la figure, peuvent connecter électriquement le substrat d'interconnexion 101 à des circuits électroniques externes à la sonde, pour contrôler les puces élémentaires 103.

Pour réaliser une telle sonde, une possibilité consiste à fixer et connecter électriquement sur une face de connexion du substrat d'interconnexion 101, une puce monolithique unique comportant l'ensemble des transducteurs ultrasonores de la sonde, puis à découper la puce en une pluralité de puces élémentaires 103 comportant chacune un ou plusieurs transducteurs ultrasonores. A l'issue de l'étape de découpe, chaque puce élémentaire 103 reste fixée et connectée électriquement au substrat d'interconnexion 101. Le substrat 101 peut alors être enroulé autour d'un cylindre, pour donner la forme souhaitée à la sonde.

La connexion électrique entre la puce monolithique et le substrat d'interconnexion 101 peut être réalisée au moyen de fils conducteurs reliant la face de connexion du substrat d'interconnexion à la face avant de la puce, opposée au substrat d'interconnexion. Une résine d'encapsulation, par exemple une résine époxy, peut être prévue pour protéger les fils de connexion. Cette méthode de connexion par fils conducteurs, aussi appelée «wire bonding» en anglais, présente toutefois certains inconvénients. En particulier, les fils conducteurs et, le cas échéant, la résine d'encapsulation, conduisent à augmenter le diamètre de la sonde. En outre, il existe un risque d'endommager les fils conducteurs lors de l'étape de découpe de la puce monolithique en puces élémentaires 103.

Alternativement, la puce monolithique et le substrat d'interconnexion 101 peuvent être assemblées par une méthode dite de montage en surface (« flip chip » en anglais) . Dans ce cas, la puce comprend des plots de connexion électrique du côté de sa face arrière, c'est à dire sa face opposée à la face d'émission/réception des ondes ultrasonores. Ces plots sont positionnés directement sur et en contact avec des plots de connexion électrique correspondant situés sur la face de connexion du substrat d'interconnexion 101. Ainsi, l'encombrement est réduit par rapport à une connexion par fils conducteurs, et les risques d'endommager les connexions électriques lors de l'étape de découpe de la puce monolithique sont réduits. Toutefois, un inconvénient de cette solution est que le coût de fabrication de la puce de transducteurs est relativement élevé, du fait de la nécessité d'intégrer des vias conducteurs traversant le substrat de la puce pour relier les transducteurs ultrasonores aux plots de connexion électrique de face arrière de la puce. Ceci est tout particulièrement vrai dans le cas de transducteurs en technologie MEMS (de l'anglais "Micro Electro Mechanical System" - microsystème électromécanique).

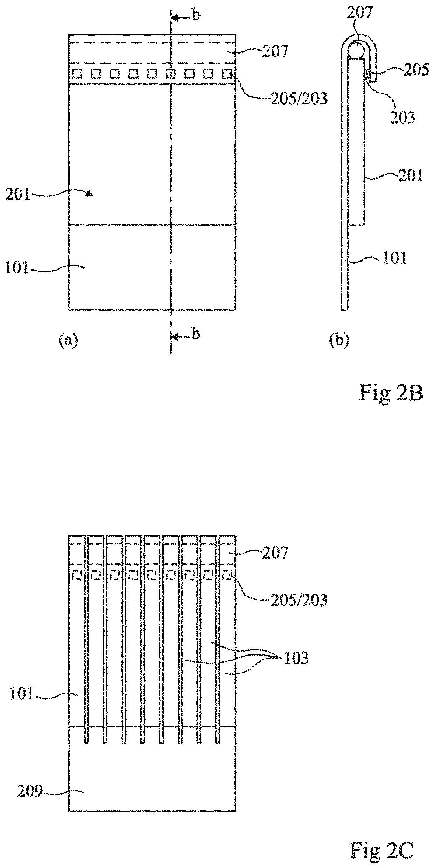

Les figures 2A, 2B et 2C illustrent des étapes successives d'un exemple d'un procédé de fabrication d'une sonde ultrasonore selon un mode de réalisation.

La figure 2A illustre l'assemblage obtenu à l'issue d'une étape de report d'une puce monolithique 201 sur une face de connexion d'un substrat d'interconnexion flexible 101.

La figure 2A comprend une vue de face (a) de l'assemblage, et une vue en coupe (b) selon le plan b-b de la vue (a).

A ce stade du procédé, le substrat d'interconnexion flexible est déroulé et positionné par exemple sur un support plan, non représenté.

La puce 201 a par exemple une forme rectangulaire. La puce 201 comprend une pluralité de transducteurs ultrasonores correspondant à l'ensemble des transducteurs de la sonde. Les transducteurs (non détaillés sur les figures) sont par exemple des transducteurs de type CMUT (transducteurs capacitifs à membrane) ou des transducteurs de type PMUT (transducteurs piézoélectriques à membrane). Les modes de réalisation décrits ne se limitent toutefois pas à ce cas particulier et s'appliquent plus généralement à tous types de transducteurs ultrasonores. La réalisation de la puce 201 n'a pas été détaillée, les modes de réalisation décrits état compatibles avec tous ou la plupart des procédés connus de fabrication d'une puce de transducteurs ultrasonores, ou la réalisation d'une telle puce étant à la portée de la personne du métier à partir des indications de la présente description. La puce 201 comprend une face avant correspondant à la face d'émission/réception des ondes ultrasonores, et une face arrière opposée à sa face avant. La puce 201 comprend des plots de connexion électrique 203 disposés du côté de sa face avant.

Le substrat d'interconnexion 101 comprend une face de connexion comportant des plots de connexion électrique 205 destinés à être connectés respectivement aux plots de connexion électrique 203 de la puce 201.

Dans cet exemple, lors du report de la puce 201, la face arrière de la puce 201 est tournée vers la face de connexion du substrat d'interconnexion 101. La face arrière de la puce 201 est par exemple fixée à la face de connexion du substrat d'interconnexion 101 par une couche adhésive, non visible sur la figure. Une partie du substrat d'interconnexion 101 comportant les plots de connexion électrique 205 n'est pas recouverte par la puce 201. Dans l'exemple représenté, le substrat d'interconnexion 101 recouvre toute la face arrière de la puce. Ainsi, dans cet exemple, la surface du substrat d'interconnexion 101 est supérieure à la surface de la puce 201. Les modes de réalisation décrits ne se limitent toutefois pas à ce cas particulier. A titre de variante, le substrat d'interconnexion 101 peut revêtir une partie seulement de la face arrière de la puce 201.

Les plots de connexion électrique 205 du substrat d'interconnexion 101 sont disposés selon un agencement sensiblement identique à celui des plots de connexion électrique 203 de la puce 201. Les plots de connexion électrique 205 sont disposés dans une partie du substrat d'interconnexion 101 destinée à être repliée sur la face avant de la puce 201, de façon à mettre en vis à vis respectivement les plots 203 et 205.

Dans l'exemple représenté, une tige 207 de forme générale cylindrique, par exemple à section circulaire, est disposée sur la face de connexion du substrat d'interconnexion 101, par exemple accolée à un bord de la puce 201. La tige 207 est disposée, en vue de dessus, entre un bord de la puce 201 et la partie du substrat d'interconnexion 101 comportant les plots de connexion 205, le long de l'axe de repliement du substrat 101. La tige 207, optionnelle, permet avantageusement de contrôler le rayon de courbure du substrat d'interconnexion 101 lors du repliement de ce dernier sur la face avant de la puce 201, limitant le risque de rupture des pistes métalliques du substrat 101 lors du pliage. La tige 207 est par exemple un fil, par exemple en nylon.

La figure 2B illustre l'assemblage obtenu à l'issue d'une étape de repli de la partie du substrat d'interconnexion 101 comportant les plots de connexion 205 sur la face avant de la puce 201, de manière à positionner les plots de connexion 205 du substrat en vis à vis des plots de connexion 203 de la puce.

La figure 2B comprend une vue de face (a) de l'assemblage, et une vue en coupe (b) selon le plan b-b de la vue (a).

Dans cet exemple, le substrat est enroulé autour de la tige 207 lors de l'étape de pliage. Un outil de forme complémentaire à la tige 207, optionnel et non représenté sur la figure 2A et 2B, peut être utilisé pour plaquer et maintenir en position le substrat 101 autour de la tige 207 lors de cette étape et des suivantes.

Lors de cette étape, les plots de connexion 205 du substrat sont fixées et connectés électriquement respectivement aux plots de connexion 203 correspondants de la puce 201. La fixation et la connexion électrique des plots 203 aux plots 205 peut être réalisée par toute méthode adaptée, par exemple par soudure, brasure, collage au moyen d'une pâte ou colle conductrice, au moyen d'un film adhésif conducteur anisotrope (ACF), etc.

De préférence, le substrat d'interconnexion flexible 101 ne recouvre pas la totalité de la face avant de la puce 201. En particulier, la surface d'émission/réception d'ultrasons de la puce n'est de préférence pas recouverte par le substrat 101, de façon à ne pas perturber la propagation des ondes ultrasonores. A titre de variante, la face avant de la puce 201 est entièrement recouverte par le substrat d'interconnexion 101 à l'issue de l'étape de pliage.

La figure 2C est une vue de face illustrant l'assemblage obtenu à l'issue d'une étape de découpe de la puce en une pluralité de puces élémentaires 103 comportant chacune un ou plusieurs transducteurs ultrasonores. Chaque puce élémentaire 103 comporte au moins un plot de connexion électrique 203 connecté à un plot de connexion électrique correspondant 205 du substrat d'interconnexion flexible 101.

La découpe peut être effectuée par toute méthode connue de découpe d'une puce en une pluralité de puces élémentaires, par exemple à l'aide d'une scie ou par découpe laser.

Dans cet exemple, les puces élémentaires 103 sont singularisées selon des lignes de découpe parallèles à l'axe central de la sonde ultrasonore cylindrique finale.

Au moins une bande 209 du substrat d'interconnexion flexible 101 n'est pas découpée lors de cette étape. A l'issue de l'étape de découpe, les puces élémentaires 103 restent reliées mécaniquement les unes aux autres par ladite bande 209.

Dans l'exemple représenté, la puce 201, la tige 207 (optionnelle) et le substrat d'interconnexion 101 sont entièrement découpés le long des chemins de découpe. Ainsi, la bande non découpée 209 est située en dehors du vis-à-vis de la puce 201. A titre de variante (non représentée), seules la partie du substrat d'interconnexion 101 recouvrant la face avant de la puce 201, la tige 207 (optionnelle) et la puce 201 sont découpées. Autrement dit, la partie du substrat d'interconnexion disposée du côté de la face arrière de la puce 201 n'est pas découpée et forme la bande de maintien mécanique des puces élémentaires 103.

A l'issue de l'étape de découpe, le substrat 101 peut être enroulé pour former une sonde cylindrique du type décrit en relation avec la figure 1.

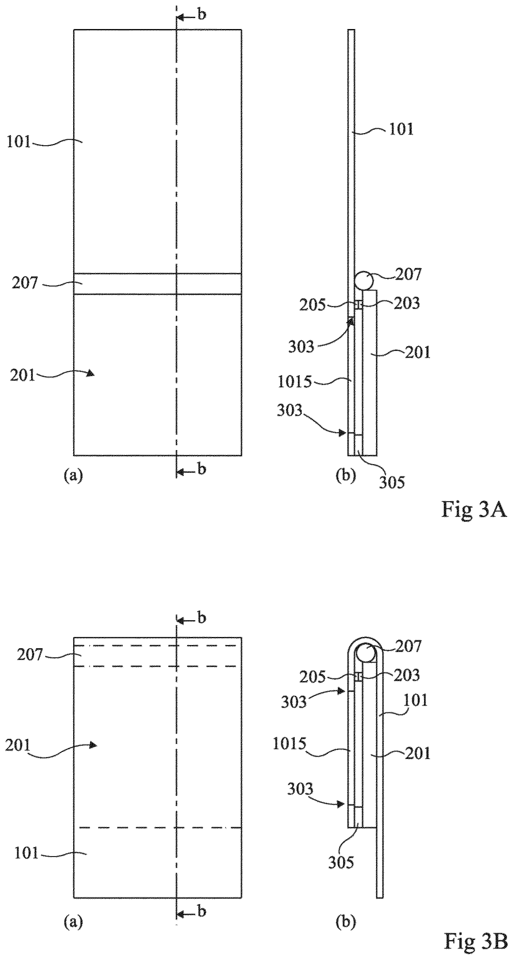

Les figures 3A et 3B illustrent des étapes successives d'un autre exemple d'un procédé de fabrication d'une sonde ultrasonore selon un mode de réalisation.

La figure 3A illustre l'assemblage obtenu à l'issue d'une étape de report d'une puce monolithique 201 sur une face de connexion d'un substrat d'interconnexion flexible 101.

La figure 3A comprend une vue de face (a) de l'assemblage, et une vue en coupe (b) selon le plan b-b de la vue (a).

L'étape de la figure 3A diffère de l'étape de la figure 2A essentiellement en ce que, dans l'exemple de la figure 3A, lors du report, la puce 201 a sa face avant tournée vers la face de connexion du substrat d'interconnexion flexible 101.

Ainsi, les plots de connexion électrique 203 de la puce sont directement mis en vis à vis des plots de connexion électrique 205 du substrat 101 et connectés électriquement aux plots 205.

Un avantage de cette variante est que la connexion des plots 203 et 205 est plus facile à réaliser lorsque le substrat flexible 101 est entièrement déroulé et plan, qu'après repliement du substrat 101 tel que décrit dans l'exemple précédent. Ceci rend en particulier plus aisée l'application d'une pression sur les plots lors de la fixation.

Dans l'exemple représenté, la face avant de la puce 201 est entièrement recouverte par le substrat 101. Dans cet exemple (non limitatif), une partie 101S du substrat 101 recouvrant la zone d'émission/réception ultrasonore de la puce 201, appelée partie sacrificielle, est prédécoupée selon une ligne de découpe 303, de façon à permettre son retrait lors d'une étape ultérieure.

Par ailleurs, dans l'exemple représenté (non limitatif), le substrat 101 comprend, par exemple dans sa partie sacrificielle 101S, une sur-épaisseur 305 en saillie en direction de la puce 201. La sur-épaisseur 305 présente par exemple une hauteur sensiblement égale à la hauteur cumulée des parties en saille des plots de connexion 203 et 205. Lors de l'étape de fixation des plots 203 de la puce sur les plots 205 du substrat, la puce 201 prend appui, par sa face avant sur la sur-épaisseur 305. Ceci permet de compenser la hauteur des plots et de maintenir la puce 201 sensiblement parallèle au substrat 101 lors de cette étape.

La figure 3B illustre l'assemblage obtenu à l'issue d'une étape de repli du substrat d'interconnexion 101 sur la face arrière de la puce 201.

Dans cet exemple, le substrat est enroulé autour de la tige 207 (optionnelle) lors de l'étape de pliage.

La face arrière de la puce 201 est par exemple fixée à la face de connexion du substrat d'interconnexion 101 par une couche adhésive, non visible sur la figure.

A l'issue de cette étape, la partie sacrificielle 101S du substrat d'interconnexion 101, et, le cas échéant, la sur-épaisseur 305, peuvent être retirées du côté de la face avant de la puce.

La suite du procédé est par exemple identique ou similaire à ce qui a été décrit précédemment.

Divers modes de réalisation et variantes ont été décrits. La personne du métier comprendra que certaines caractéristiques de ces divers modes de réalisation et variantes pourraient être combinées, et d'autres variantes apparaîtront à la personne du métier. En particulier, les modes de réalisation décrits ne se limitent pas à l'exemple particulier décrit ci-dessus d'application à une sonde ultrasonore cylindrique, mais peuvent être appliqués à d'autres formes de sonde non planes, moyennant d'éventuelles adaptations à la portée de la personne du métier.

En outre, les modes de réalisation décrits ne se limitent pas à l'exemple d'application indiqué ci-dessus à la réalisation de sondes ultrasonores intravasculaires, mais s'appliquent plus généralement à la réalisation de tous types de sondes ultrasonores de formes non planes, par exemple de petites dimensions, pour des applications médicales ou non-médicales.

Par ailleurs, bien que l'on ait décrit ci-dessus des exemples de réalisation dans lesquels le substrat flexible 101 est replié sur la face avant ou sur la face arrière de la puce monolithique 201 avant découpe de la puce 201 en puce élémentaires 103, les modes de réalisation décrits ne se limitent pas à ce cas particulier. A tire de variante, la découpe de la puce monolithique 201 peut être réalisée après la fixation de cette dernière sur le substrat d'interconnexion flexible 101 mais avant l'étape de repliement d'une partie du substrat 101 sur la face de la puce opposée à sa face de report. En particulier, la personne du métier saura adapter l'exemple des figures 2A à 2C en mettant en oeuvre la découpe de la puce 201 entre l'étape de report de la figure 2A et l'étape de pliage de la figure 2B. De façon similaire, la personne du métier saura adapter l'exemple des figures 3A et 3B en mettant en oeuvre la découpe de la puce 201 entre l'étape de report de la figure 3A et l'étape de pliage de la figure 3B.

Par ailleurs, dans les deux exemples de mise en œuvre décrits en relation avec les figures, on a décrit des connexions uniquement en face avant (masse et point chaud de chaque puce). Il est toutefois possible de prévoir des connexions électriques sur les deux faces principales de chaque puce.

A titre d'exemple, une connexion à la masse peut être prévue en face avant, et reportée sur la partie repliée du substrat souple. Des électrodes de connexion aux transducteurs ultrasonores peuvent être prévues en face arrière, connectées à respectivement à des plots correspondants du substrat flexible.

Selon un autre exemple, une connexion à la masse peut être prévue en face arrière, connectée à un plan métallique, par exemple en cuivre sur le substrat d'interconnexion flexible. Des électrodes de connexion aux transducteurs ultrasonores peuvent être prévues en face avant, connectées sur la partie repliée du substrat d'interconnexion flexible.

## Revendications

1. Procédé de fabrication d'une sonde ultrasonore, comprenant les étapes suivantes :
a) rapporter, sur une face d'un substrat d'interconnexion flexible (101), une puce (201) comportant une pluralité de transducteurs ultrasonores, la puce (201) comportant une face avant comportant au moins un plot de connexion électrique (203) et une face arrière ;
b) replier une partie du substrat d'interconnexion flexible (101), de façon que, à l'issue de l'étape b), ladite face du substrat d'interconnexion flexible (101) s'étende et soit fixée sur au moins une partie de la face arrière de la puce et sur au moins une partie de la face avant de la puce, ledit au moins un plot de connexion électrique (203) de la face avant de la puce étant connecté à au moins un plot de connexion électrique (205) correspondant de ladite face du substrat d'interconnexion (101) ; et
c) découper la puce (201) en une pluralité de puces élémentaires (103) comportant chacune un ou plusieurs transducteurs ultrasonores.

2. Procédé selon la revendication 1, dans lequel l'étape c) est mise en oeuvre après l'étape b).

3. Procédé selon la revendication 1, dans lequel l'étape c) est mise en oeuvre avant l'étape b).

4. Procédé selon l'une quelconque des revendications 1 à 3, dans lequel, à l'étape a), la puce (201) a sa face arrière tournée vers la face du substrat d'interconnexion (101) comportant ledit au moins un plot de connexion électrique (205) du substrat d'interconnexion (101).

5. Procédé selon l'une quelconque des revendications 1 à 3, dans lequel, à l'étape a), la puce (201) a sa face avant tournée vers la face du substrat d'interconnexion (101) comportant ledit au moins un plot de connexion électrique (205) du substrat d'interconnexion (101).

6. Procédé selon l'une quelconque des revendications 1 à 5, dans lequel, à l'issue de l'étape c), les puces élémentaires (103) restent reliées mécaniquement les unes aux autres par une bande (209) non découpée du substrat d'interconnexion flexible (101).

7. Procédé selon l'une quelconque des revendications 1 à 6, dans lequel, lors de l'étape b), une tige cylindrique (207) est positionnée sur le substrat d'interconnexion (101) pour contrôler la courbure du substrat d'interconnexion (101) lors du pliage.

8. Procédé selon l'une quelconque des revendications 1 à 7, comprenant, après les étapes a), b) et c), une étape de courbure du substrat d'interconnexion (101) selon une forme souhaitée de la sonde, par exemple une forme cylindrique.

9. Procédé selon l'une quelconque des revendications 1 à 8, dans lequel les transducteurs ultrasonores sont des transducteurs de type CMUT ou PMUT.

10. Procédé selon l'une quelconque des revendications 1 à 9, dans lequel la puce (201) comporte en outre au moins un plot de connexion électrique du côté de sa face arrière.

11. Sonde ultrasonore comportant un substrat d'interconnexion flexible (101) et une pluralité de puces élémentaires (103) fixées à une face du substrat d'interconnexion flexible (101), chaque puce élémentaire (103) comportant un ou plusieurs transducteurs ultrasonores, chaque puce élémentaire (103) comportant une face avant comportant au moins un plot de connexion électrique (203) et une face arrière,
dans laquelle une partie du substrat d'interconnexion flexible (101) est replié sur les puces élémentaires (103), de façon que ladite face du substrat d'interconnexion flexible (101) s'étende et soit fixée sur au moins une partie de la face arrière de chaque puce élémentaire (103) et sur au moins une partie de la face avant de chaque puce élémentaire (103), ledit au moins un plot de connexion électrique (203) de la face avant de chaque puce élémentaire (101) étant connecté à un plot de connexion électrique (205) correspondant de ladite face du substrat d'interconnexion (101).
